# NEUE EUROPÄISCHE PATENTSCHRIFT

(11) **EP 0 167 575 B2**
(45) Veröffentlichungstag und Bekanntmachung des Hinweises auf die Entscheidung über den Einspruch: **27.10.1999**
(45) Hinweis auf die Patenterteilung: 26.05.1993
(21) Anmeldenummer: 85900447.5
(22) Anmeldetag: 21.12.1984
(51) Int. Cl.: C07K 14/58, C07K 7/08, A61K 38/17

(54) **CARDIODILATIN, NEUES PEPTIDHORMON UND VERFAHREN ZU SEINER HERSTELLUNG**
CARDIODILATINE, A PEPTIDE HORMONE AND PRODUCTION PROCESS THEREOF
CARDIODILATINE, UNE HORMONE PEPTIDIQUE ET SON PROCEDE DE FABRICATION

(30) Priorität: 24.12.1983 DE 3346953
(43) Veröffentlichungstag der Anmeldung: 15.01.1986
(73) Patentinhaber: FORSSMANN, Wolf-Georg, Prof. Dr. med., D-69121 Heidelberg (DE)
(72) Erfinder: FORSSMANN, Wolf-Georg, D-6900 Heidelberg (DE)
(74) Vertreter: Werner, Hans-Karsten, Dr.Dipl.-Chem.
(86) Internationale Anmeldenummer: DE8400279
(87) Internationale Veröffentlichungsnummer: WO8502850

(56) Entgegenhaltungen:
- CHEMICAL ABSTRACTS, Band 102, 21 Jan 1985, Columbus, OH (US); K.NAKAYAMA et al., Seite 166, Nr. 18640t#
- CHEMICAL ABSTRACTS, Band 101, 22 Okt 1984, Columbus, OH (US); N.G. SEIDAH et al., Seite 137, Nr. 144780f#
- BIOCHEM. AND BIOPHYS. RESEARCH COMMUNICATIONS, Band 118, Nr. 1, 1984; Seiten 131-139#
- SCIENCE, Vol. 221, S. 71-73 (01-07-1983)
- LIFE SCIENCES, Vol. 33, S. 297-302 (1983)

## Beschreibung

Die Erfindung betrifft ein neues Peptidhormon und ein Verfahren zu seiner Herstellung.

Aus elektronenmikroskopischen Untersuchungen war es bekannt, daß der rechte Herzvorhof (Atrium) des Schweines zwei verschiedene Zelltypen enthält, von denen einer die Morphologie endokriner Zellen (Sekretgranular) aufweist. Durch morphologische und histochemische Untersuchungen des rechten Atriums konnte jedoch keine Hormonsubstanz, wie die des bereits bekannten Typus in myoendokrinen Zellen des Herzvorhofes (Aurikel) bestimmt werden. Dagegen wurden viele der bekannten Neuropeptidhormone in Herznerven festgestellt.

Es wurde nun gefunden, daß Atrium-Extrakte (Herzvorkammer) neben einer schon beschriebenen diuretischen Aktivität (vgl. J.-P. Marie, H. Guillemont, P.Y. Halt. Pathol. Biol. 24 (1976) 549-554), überraschenderweise Effekte auf die Inotropie des Herzmuskels selbst bzw. Einflüsse auf die glatte Gefäßmuskulatur, sowie Einflüsse auf die Schweißsekretion aufweisen. Weiter wurde gefunden, daß diese biologischen Wirkungen durch ein neues Peptidhormon verursacht werden, dem im Hinblick auf seine Wirkungen eine große klinische und therapeutische Bedeutung zukommt, insbesondere im Hinblick auf die Diagnostik und Therapie der Hypertonie.

Gegenstand der Erfindung ist deshalb, das neue Peptidhormon Cardiodilatin mit der N-terminalen Aminosäuresequenz: 
Asn-Pro-Val-Tyr-Gly-Ser-Val-Ser-Asn-Ala-Asp-Leu-
Met-Asp-Phe-Lys-Asn-Leu-Leu-Asp-His-Leu-Glu-Asp-

Lys-Met-Pro-Leu-Glu-Asp-Glu-Ala-Met-Pro-Pro-Gln-
Val-Leu-Ser-Glu-Gln-Asp-Glu ...,
 bei dem auch in der nachfolgenden Aminosäuresequenz Argininreste vorhanden sind und/oder seiner biologisch aktiven Fragmente, erhältlich in Form aktiver Fraktionen, ausgehend von Atriumextrakt von Schweineatrien, durch Fraktionierung eines mit verdünnter Säure extrahierten gekochten Atriummaterials gemäß V. Mutt, Arkiv Kemi 15, 69 - 74 (1959) nach üblichen biochemischen Reinigungsmethoden, unter Anwendung eines Tests, bei dem die aktiven Fraktionen durch die relaxierende Wirkung auf Arteria renalis (Kaninchen), Aorta abdominalis (Kaninchen, Ratte) und Arteria mesenterica inferior (Kaninchen) im Organbad bestimmt werden.

Außerdem wurde gefunden, daß auch Fragmente, insbesondere solche, deren Aminosäuresequenzen durch die Sequenzen zwischen Methionin - un/oder Argininresten der Cardiodilatinsequenz bestimmt sind, die biologischen Aktivitäten des Cardiodilafins, wenn auch zum Teil in abgeschwächter Form, aufweisen.

Gegenstand der Erfindung sind deshalb auch solche Fragmente, deren Aminosäuresequenzen durch die Sequenzen zwischen Methionin - und/oder Argininresten der Cardiodilatinsequenz bestimmt sind, wie z. B. das Fragment mit der Aminosäuresequenz Asp - Phe - Lys - Asn - Leu - Leu - Asp - His - Leu - Glu - Asp - Lys - Hse (1) oder das Fragment mit der Aminosäuresequenz Pro - Leu - Glu - Asp - Glu - Ala - Hse (2), und das N - terminale Fragment Asn - Pro - Val - Tyr - Gly - Ser - Val - Ser - Asn - Ala - Asp - Leu - Hse sowie die jeweils C-terminal verbleibenden Rumpfsequenzen des Cardiodilatin, verkürzt um die aufgeführten Homoserin - Peptide. Diese Fragmente erhält man durch partielle Bromcyanspaltungen, wobei die N- terminale Verkürzung des Cardiodilatin zu abgelösten Fragmenten führt, die Jeweils C-terminal mit Homoserin (Hse) enden. Auch die durch Spaltung mit Arginin - spezifischer Endopeptidase gebildeten, N - terminal verkürzten Fragmente des Cardiodilatin wiesen qualitativ die gleiche biologische Wirksamkeit wie das Stamm - Molekül auf, wenn auch in abgeschwächter Form.

Die hergestellten Fragmente weisen eine interessante biologische Aktivität auf und zwar eignen sie sich zur Bildung von Antikörpern, welche das gesamte Cardiodilatin zu erkennen vermögen und daher auch zu dessen Nachweis im Rahmen eines Immunassays verwendet werden können. Geeignet sind sowohl die zahlreichen bekannten Ausführungsformen des RIA (Radio-Immun-Assay) als auch des EIA (Enzym-Immun-Assay).

Erfindungsgemäß wird Cardiodilatin gewonnen, indem man Atriumextrakt als Ausgangsmaterial verwendet und das mit wässrigen Lösungsmitteln extrahierbare Material nach üblichen biochemischen Reinigungsmethoden fraktioniert unter Anwendung eines Tests, bei dem die aktive Fraktion durch die relaxierende Wirkung auf glatte Muskulatur bestimmt wird sowie durch Hydrometrie die Leitfähigkeitsmessung der Wasserabgabe durch die Haut. Die Reindarstellung des Peptidhormons Cardiodilatin erfolgt aus Atrium-Extrakten von Schweineatrien (V. Mutt, Arkiv Kemi 15, 69 -74 (1959). Vorzugsweise wird Cardiodilatin hergestellt, indem man gekochtes Atriummaterial mit verdünnter Säure extrahiert. Als verdünnte Säure eignen sich vor allem die Carbonsäuren. aber auch Mineralsäuren können eingesetzt werden. Bevorzugt verwendet man Essigsäure, besonders bevorzugt in einer Konzentration von 0.1 bis 0.3 M.

Die weitere Reinigung kann erfindungsgemäß so erfolgen, das man den Säureextrakt an Alginsäure adsorbiert, die Peptide mit verdünnter Mineralsäure wieder von der Algensäure eluiert, das Eluat einer Salzfällung unterwirft, den dabei gebildeten Niederschlag auflöst und erneut mit Ethanol fällt. Der Ethanolniederschlag wird dann aufgelöst und über Carboxymethylcellulose chromatographiert. Ein so gewonnenes Material ist in der Regel für pharmazeutische oder analytische Zwecke ausreichend rein. Der oben beschriebene, durch Salzfällung gewonnene Niederschlag kann alternativ auch direkt entsalzt und gereinigt werden durch Gelchromatographie, vorzugsweise an Sephadex G 25.

Eine Feinreinigung ist möglich mittels Hochdruck - Flüssigkeits - Chromatographie (HPLC) an einem Umkehrphasen - Kieselgel (Gradientenelution: 0,1% Trifluoressigsäure in Wasser gegen Acetonitril, 0 bis 60%) und Abtrennen der aktiven Fraktionen.

Zweckmäßigerweise wird der HPLC an einem Umkehrphasen - Kieselgel eine HPLC an einer lonenaustauschersäule vorgeschaltet und/oder die HPLC an Umkehrphasen - Kieselgel ein - oder mehrmals, vorzugsweise zweimal, wiederholt.

Die N-terminalen Fragmente, welche entweder synthetisch oder durch Spaltung am N-terminalen Methioninrest oder Argininrest erhalten werden, bewirken nach Konjugation mit einer Trägerprotein Anti - körperbildung bei Verabreichung an andere Tierarten wie Kaninchen oder Mäuse (Balb C), weisen also auch Hapteneigenschaften auf. Diese Antikörper können als spezifischer Ligand in der Affinitätschromato - graphie zur Anreicherung (Isolierung) und zum Nachweis des Peptidhormons Cardiodilatin eingesetzt werden. Die Affinitätschromatographie mit einem N - terminalen Fragment - Antikörper als spezifischer Ligand stellt somit ein weiteres geeignetes Verfahren zur Anreicherung (Isolierung) des Peptidhormons dar, mit dem Cardiodilatin meist schon in ausreichend reiner Form erhalten werden kann. Die Affinitätschromatographie kann auch mit einer der vorstehend angeführten chromatographischen Verfahren zur Isolierung des Peptidhormons angewendet werden, oder aber es kann sich an eine der chromatographischen Methoden eine weitere Reinigung nach einem oder mehreren dafür geeigneten Verfahren anschließen: geeignete Verfahren sind z. B: Elektrophorese, Fällung. Adsorptions-Chromatographie, Ionenaustausch-Chromatographie, Affinitäts - Chromatographie, isoelektrische Fokusierung, oder eine Kombination einer oder mehrerer gleicher oder verschiedener dieser Schritte.

Die sich zwischen zwei Met-Resten der Peptidkette befindlichen Fragmente lassen sich aus dem Cardiodilatin auf an sich bekannte Weise durch Spaltung mit geeigneten Hydrolasen herstellen, die Fragmente lassen sich aber auch, wie z. B. die oben angegebenen Fragmente mit der Aminosäuresequenz (1) und (2) oder N-terminale Fragmente, auf synthetischem Wege durch an sich bekannte Peptidsynthesen herstellen, wie z. B. nach der Methode von Merrifield oder Synthese mit gemischten Anhydriden in Lösung.

Als differenzierender Bioassay für die Wirkung des Peptides wird der relaxierende Effekt auf Arteria renalis (Kaninchen), Aorta abdominalis (Kaninchen, Ratte) und Arteria mesenterica inferior (Kaninchen) im Organbad benutzt, wobei die nach Adrenalin -Vorbehandlung kontrahierten Gefäßmuskelstreifen nach Zugabe des Cardiodilatins eine deutliche Relaxation zeigen. Diese Relaxation des Muskelstreifens aus Arteria renalis ist bei geringen Dosen von ca. 10 ng bis 100 ng/10 ml Organbad nachweisbar. Bei Streifen der Aorta als Testmuskel zeigte sich bei einer ca. 10fach höheren Dosis eine nachweisbare Relaxation, während an der Arteria mesenterica inferior des Kaninchens kaum eine Aktivität beobachtet wurde.

Diese biologischen Wirkungen zeigen, daß das Peptidhormon Cardiodilatin und, wie gefunden wurde, auch seine Fragmente, insbesondere die nach oder zwischen Met- Resten stehen und nach partieller Bromcyanspaltung gewonnen werden können, eine große klinische (diagnostische) und therapeutische Bedeutung besitzen, und zwar insbesondere hinsichtlich:
1. Differenzierte Vasodilatation bestimmter Gefäßbetten,
2. Diagnostik der Hypertonie,
3. Therapie der Hypertonie,
4. Möglichkeit, Vorhofsdilatationen aufgrund der Freigabe des Hormons zu diagnostizieren, und
5. Anwendung dieses Peptidhormones als Substitution bei Patienten, denen künstliche Herzen implantiert wurden.
6. Desweiteren wird eine synchrone Regulation des Blutvolumens und der Blut - Elektrolyte vermutet, auf die das Peptid Einfluß hat.
7. Hauterkrankungen, insbesondere mit Störung der Schweißsekretation,
8. den cardiovasculären Schock,
9. Erkrankungen der Nieren und Nebennierenrinde
10. Erkrankungen des Verdauungstraktes, insbesondere von Motilitätsstörungen.

Gegenstand der Erfindung sind deshalb auch Arzneimittel zur Verwendung bei den oben angegebenen Diagnostizier- und Therapieverfahren, die das neue Peptidhormon Cardiodilatin und/oder wirksame Fragmente davon enthalten. Die Arzneimittel können in den üblichen Anwendungsformen zur oralen oder parenteralen Applikation vorliegen, wie z. B. als Tabletten, Suppositorien, Dragees, Lösungen usw., gegebenenfalls zusammen mit üblichen, pharmakologisch verträglichen Träger- und/oder Verdünnungsmitteln. Die Menge an Cardiodilatin beträgt vorzugsweise 10 bis 1000 ng/Dosiseinheit.

### Beispiel 1

Unter Verwendung konventioneller elektronenmikroskopischer Methoden wurden diejenigen Stellen im rechten Atrium von Schweineherzen bestimmt, die die höchste Dichte an myoendokrinen Zellen (endokrine Sekretgranular) aufwiesen. Diese Zellen wurden hauptsächlich an den dünnwandigen Stellen des Aurikels gefunden. Im Hinblick auf ihre Größenverteilung (230 und 50 nm) und ihre Elektronendichte sind diese Schweinegranula mit den menschlichen Granula vergleichbar; die Zahl der Granula beträgt ca. 0,02 bis 0,04 Granula/µm² der Schnittfläche.

Dieses oben beschriebene Gewebe des rechten Atriums wurde 20.000 Schweineherzen entnommen und nach bekannten Verfahren extrahiert (vgl. V. Mutt, Arkiv Kemi 15, 69-74 (1959), S.I. Said und V. Mutt, Eur. J. Biochem. 28, 199-204 (1972), V. Mutt Gut hormones, S. 21-27, S.R. Bloom(editor) Edinburgh-London - New York: Churchill Livingstone, 1978).

### Beispiel 2

Hierzu wurden 40 kg Schweineartriumgekocht und mit 0,2 M Essigsäure extrahiert. Das Filtrat wurde an Alginsäure adsorbiert, die Algensäure mit Ethanol gewaschen und anschließend mit 0,2 M Salzsäure eluiert. Das die Peptide enthaltende Eluat wird dann mit Salz versetzt, bis die Peptide ausfallen und der Niederschlag abzentrifugiert. Der Niederschlag wird wieder in Phosphatpuffer, pH 7 aufgelöst und durch Zusatz von Ethanol erneut gefällt. Der Niederschlag wurde erneut aufgelöst in Phosphatpuffer, pH 6,4 und auf eine Carboxymethylcellulose aufgezogen. Die Säule war vorher mit einem Puffer, enthaltend 0,03 M NaOH, 0,025 M H₃PO₄, 0,5 % Thiodiglycol, pH 6,4, äquilibriert worden. Nach Elution mit dem gleichen Puffer erfolgte eine weitere Elution mit einem Puffer enthaltend 0,03 M NaOH, 0,025 M H₃PO₄ und 0,2 M NaCI PH 6,4. Hierbei wurden die aktivsten Fraktionen eluiert.

Die mit dem salzhaltigen Puffer eluierte Fraktion wird auf eine lonenaustauschersäule (TSK - Carboxymethylcellulose der Firma LKB) gegeben und mit 0,03 M Natriumphosphatpuffer, pH 6,4 und einem Natriumchloridgradienten von 0 bis 0,5 M eluiert. Die aktive Fraktion wird gesammelt, über eine Molekularsiebsäule (Sephadex G 25^{(R)}) entsalzt und der präparativen Hochdruckflüssigkeitschromatographie (HPLC) mit umgekehrter Phase unterworfen, vorzugsweise unter Verwendung einer Organogen C₁₈ - 5µ ^{R} - Säule.
- Säule:: Waters C₁₈ - µ Bondapak, 4 x 250 nm.
- Gradientenelution:: 0,1% Trifluoressigsäure in Wasser gegen Acetonitril, 0 bis 60 % in 45 Minuten.

Das Peptid Cardiodilatin tritt bei 45% Acetonitril aus der HPLC - Säule aus und zeigt eine Retentionszeit von 24,1 Minuten.

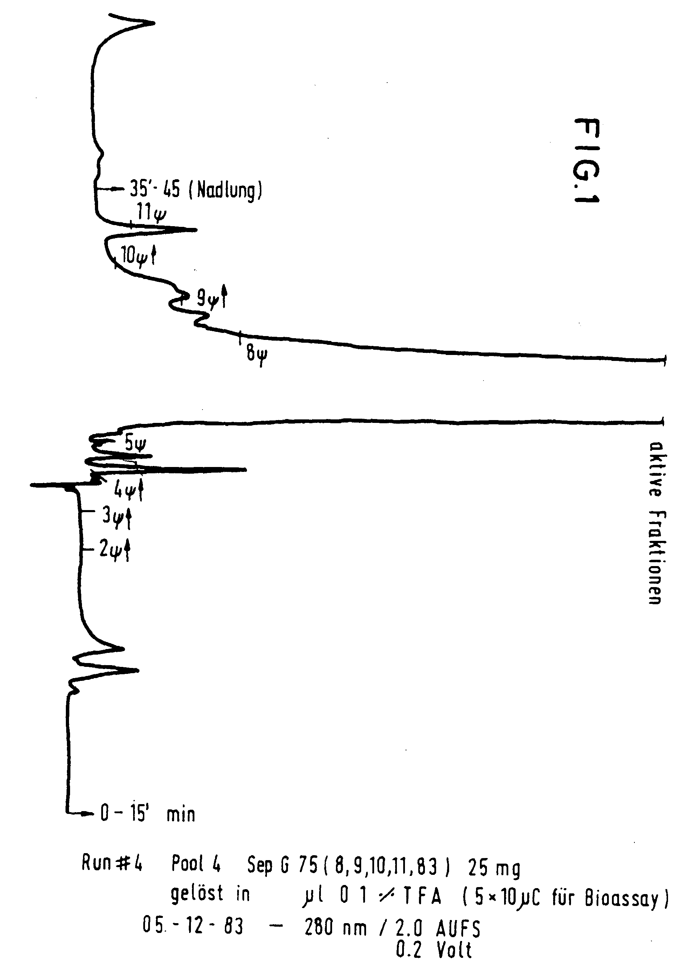

Fig. 1 zeigt eine graphische Darstellung dieser HPLC.

Die erhaltene aktive Fraktion wurde danach nochmals zweimal der gleichen HPLC unterworfen.

Der isoelektrische Punkt I.P. wurde mit 6 bis 6,5 bestimmt.

Für die verschiedenen Stufen des Reinigungsverfahrens wurden biologische Tests nach den untenstehenden Bioassays durchgeführt, wobei sich mit fortschreitendem Grad der Reinigung ein Ansteigen der spezifischen Aktivität zeigte.

### Durchführung der Bioassays:

Es wurden Bioassays an glatter Gefäßmuskulatur durchgeführt, bei der die Entspannung in einem Organbad unter Einwirkung der zu testenden Fraktionen bestimmt wurde.

## Patentansprüche

1. Peptidhormon Cardiodilatin mit der N-terminalen Aminosäuresequenz: 
Asn-Pro-Val-Tyr-Gly-Ser-Val-Ser-Asn-Ala-Asp-Leu-
Met-Asp-Phe-Lys-Asn-Leu-Leu-Asp-His-Leu-Glu-Asp-
Lys-Met-Pro-Leu-Glu-Asp-Glu-Ala-Met-Pro-Pro-Gln-
Val-Leu-Ser-Glu-Gln-Asp-Glu ...,
 bei dem auch in der nachfolgenden Aminosäuresequenz Argininreste vorhanden sind und/oder seiner biologisch aktiven Fragmente, erhältlich in Form aktiver Fraktionen, ausgehend von Atriumextrakt von Schweineatrien, durch Fraktionierung eines mit verdünnter Säure extrahierten gekochten Atriummaterials gemäß V. Mutt, Arkiv Kemi 15, 69 - 74 (1959) nach üblichen biochemischen Reinigungsmethoden, unter Anwendung eines Tests, bei dem die aktiven Fraktionen durch die relaxierende Wirkung auf Arteria renalis (Kaninchen), Aorta abdominalis (Kaninchen, Ratte) und Arteria mesenterica inferior (Kaninchen) im Organbad bestimmt werden, wobei die Aminosäuresequenz der biologisch aktiven Fragmente durch die Sequenzen zwischen Methioninresten und/oder Argininresten der Cardiodilatinsequenz bestimmt sind und/oder N-terminalen Fragmenten, erhältlich durch Spaltung mit argininspezifischer Endopeptidase.

2. Cardiodilatin-Fragment nach Anspruch 1 mit der Aminosäuresequenz

Asp-Phe-Lys-Asn-Leu-Leu-Asp-His-Leu-Glu-Asp-Lys-
Hse.

3. Cardiodilatin-Fragment nach Anspruch 1 mit der Aminosäuresequenz

Pro-Leu-Glu-Asp-Glu-Ala-Hse.

4. N-terminales Cardiodilatin-Fragment

Asn-Pro-Val-Tyr-Gly-Ser-Val-Ser-Asn-Ala-Asp-Leu-
Hse.

5. Verfahren zur Herstellung des Peptidhormons Cardiodilatin, dadurch gekennzeichnet, daß man Atriumextrakt als Ausgangsmaterial verwendet und das mit wäßrigen Lösungsmitteln extrahierbare Material nach üblichen biochemischen Reinigungsmethoden fraktioniert unter Anwendung eines Tests, bei dem die aktive Fraktion durch die relaxierende Wirkung auf glatte Muskulatur bestimmt wird, sowie durch Hydrometrie die Leitfähigkeitsmessung der Wasserabgabe durch die Haut.

6. Verfahren nach Anspruch 5, dadurch gekennzeichnet, daß man gekochtes Atriummaterial mit verdünnter Säure extrahiert.

7. Verfahren nach Anspruch 5, dadurch gekennzeichnet, daß man den- Säureextrakt an Alginsäure adsorbiert, die Peptide mit verdünnter Mineralsäure eluiert, das Eluat mit Na-acetat puffert, neutralisiert, einer Salzfällung unterwirft, den Niederschlag auflöst und einer Ethanolfällung unterwirft, den Ethanolniederschlag wieder auflöst und über Carboxymethylcellulose chromatographiert.

8. Verfahren nach Anspruch 7, dadurch gekennzeichnet, daß der zur Salzfällung gewonnene Niederschlag mittels Gelchromatographie, vorzugsweise an Sephadex G 25, direkt entsalzt und gereinigt wird.

9. Verfahren nach Anspruch 5 oder 8, dadurch gekennzeichnet, daß man den Atriumextrakt mit Essigsäure herstellt.

10. Verfahren nach einem der Ansprüche 6 bis 8, dadurch gekennzeichnet, daß man zur Feinreinigung eine Hochdruckflüssigkeits-Chromatographie (HPLC) an einem Umkehrphasen-Kieselgel (Gradientenelution: 0,1 % Trifluoressigsäure in Wasser gegen Acetonitril, 0 bis 60 %) durchführt und die aktiven Fraktionen abtrennt.

11. Verfahren nach einem der Ansprüche 6 bis 8, dadurch gekennzeichnet, daß man zur Feinreinigung Immunadsorptionschromatographie durchführt, vorzugsweise unter Anwendung immobilisierter Antikörper gegen Cardiodilatinfragmente gemäß Anspruch 1.

12. Verfahren nach Anspruch 10, dadurch gekennzeichnet, daß man der HPLC an einem Umkehrphasen-Kieselgel eine HPLC an einer Ionenaustauschersäule vorschaltet und/oder die HPLC an Umkehrphasen-Kieselgel ein- oder mehrmals wiederholt.

13. Arzneimittel, insbesondere zur Diagnose und Behandlung von Hypertonie und für Gefäß- und Herzoperationen, und Behandlung von Hypertonie, Gefäß- und Herzerkrankungen nach Gefäß- und Herzoperationen, auch Hauterkrankungen mit Störungen der Schweißsekretion, des cardiovasculären Schocks, von Erkrankungen der Nieren und Nebennierenrinde und von Erkrankungen des Verdauungstraktes, insbesondere von Motilitätsstörungen, dadurch gekennzeichnet, daß es als Wirkstoff eine Substanz nach einem der Ansprüche 1 bis 5 zusammen mit einem pharmakologisch verträglichen Träger und/oder Verdünnungsmittel enthält.

14. Arzneimittel nach Anspruch 13, dadurch gekennzeichnet, daß es 10 bis 1000 ng Cardiodilatin/Dosiseinheit enthält.

15. Verfahren zur spezifischen Bestimmung von Cardiodilatin, dadurch gekennzeichnet, daß man nach den Prinzipien der Immuntests arbeitet und einen Antikörper, der gegen Cardiodilatin nach Anspruch 1 oder gegen eines der Fragmente der Ansprüche 2 bis 5 gerichtet ist, verwendet.

## Claims

1. The peptide hormone cardiodilatin having the N-terminal amino acid sequence 
Asn-Pro-Val-Tyr-Gly-Ser-Val-Ser-Asn-Ala-Asp-Leu-
Met-Asp-Phe-Lys-Asn-Leu-Leu-Asp-His-Leu-Glu-Asp-
Lys-Met-Pro-Leu-Glu-Asp-Glu-Ala-Met-Pro-Pro-Gln-
Val-Leu-Ser-Glu-Gln-Asp-Glu ...,
 wherein arginine moieties are present also in the subsequent amino acid sequence, and/or its biologically active fragments, obtainable in the form of active fractions, starting from atrium extract from porcine atria by fractionating a boiled atrium material extracted with diluted acid according to V. Mutt, Arkiv Kemi 15, 69-74 (1959) in accordance with conventional biochemical purification methods, using an assay wherein the active fractions are determined by the relaxant effect on arteria renalis (rabbit), aorta abdominalis (rabbit, rat) and arteria mesenterica inferior (rabbit) in an organ bath, the amino acid sequence of said biologically active fragments being defined by the sequences between methionin residues and/or arginin residues of the cardiodilatin sequence and/or N-terminal fragments obtainable by cleavage with argininspecific endopeptidase.

2. The cardiodilatin fragment according to claim 1, having the amino acid sequence

Asp-Phe-Lys-Asn-Leu-Leu-Asp-His-Leu-Glu-Asp-Lys-
Hse.

3. The cardiodilatin fragment according to claim 1, having the amino acid sequence

Pro-Leu-Glu-Asp-Glu-Ala-Hse.

4. The N-terminal cardiodilatin fragment

Asn-Pro-Val-Tyr-Gly-Ser-Val-Ser-Asn-Ala-Asp-Leu-
Hse.

5. A process for preparing the peptide hormone cardiodilatin, characterized in that atrium extract is employed as the starting material, and the material capable of being extracted with aqueous solvents is fractionated according to conventional biochemical purification methods using an assay wherein the active fraction is determined by the relaxant effect onto smooth muscles, and the conductivity measurement of the release of water through the skin by hydrometry.

6. The process according to claim 5, characterized in that boiled atrium material is extracted with a diluted acid.

7. The process according to claim 5, characterized in that the acidic extract is adsorbed on alginic acid, the peptides are eluted with diluted mineral acid, the eluate is buffered with Na acetate, neutralized, subjected to salt precipitation, the precipitate is dissolved and subjected to ethanol precipitation, the ethanol precipitate is redissolved and chromatographed on carboxymethylcellulose.

8. The process according to claim 7, characterized in that the precipitate obtained by salt precipitation is directly desalted and purified by means of gel chromatography, preferably on Sephadex G 25.

9. The process according to claim 5 or 8, characterized in that the atrium extract is prepared using acetic acid.

10. The process according to any of claims 6 to 8, characterized in that, for refining purification, high-pressure liquid chromatography (HPLC) on a reversed phase silica gel is carried out (gradient elution: 0.1% trifluoroacetic acid in water against acetonitrile, from 0 to 60%), and the active fractions are separated off.

11. The process according to any of claims 6 to 8, characterized in that, for refining purification, immunoadsorbent chromatography is carried out, preferably by using immobilized antibodies against cardiodilatin fragments according to claim 1.

12. The process according to claim 10, characterized in that HPLC on an ion-exchange column is carried out prior to said reversed phase silica gel HPLC, and/or said reversed phase silica gel HPLC is repeated once or several times.

13. A medicament, especially for diagnostics and treatment of hypertension and for vascular and cardiac surgery, and for the treatment of hypertension, vessel and heart diseases after vascular and cardiac surgery, also skin diseases including perspiration disorders, cardiovascular shock, renal and adrenal cortical disorders, diseases of the gastro-intestinal tract, especially motility disorders, characterized by containing a substance according to any of claims 1 to 5 as the active ingredient in combination with a pharmacologically acceptable carrier and/or diluent.

14. The medicament according to claim 13, characterized by containing from 10 to 1000 ng of cardiodilatin per unit dose.

15. A method for the specific determination of cardiodilatin, characterized in that the principles of an immunoassay are employed, and an antibody is used which is directed against cardiodilatin according to claim 1 or against any of the fragments according to claims 2 to 5.

## Revendications

1. Hormone peptidique cardiodilatine comportant la séquence N-terminale d'amino-acides : 
Asn-Pro-Val-Tyr-Gly-Ser-Val-Ser-Asn-Ala-Asp-Leu-Met-
Asp-Phe-Lys-Asn-Leu-Leu-Asp-His-Leu-Glu-Asp-Lys-Met-
Pro-Leu-Glu-Asp-Glu-Ala-Met-Pro-Pro-Gln-Val-Leu-Ser-
Glu-Gln-Asp-Glu...,
 dans laquelle des restes d'arginine sont présents aussi dans la séquence suivante d'amino-acides, et/ou ses fragments biologiquement actifs, pouvant être obtenue sous la forme de fractions actives à partir d'un extrait d'atrium de porc, par fractionnement d'un produit d'atrium bouilli extrait avec un acide dilué conformément à V. Mutt, *Arkiv Kemi* 15, 69 - 74 (1959) selon des procédés biochimiques usuels de purification, en utilisant un test dans lequel les fractions actives sont déterminées dans un bain de l'organe au moyen de l'effet relaxant sur l'artère rénale (lapin), l'aorte abdominale (lapin, rat) et l'artère mésentérique inférieure (lapin), la séquence d'amino-acides des fragments biologiquement actifs étant définie par les séquences situées entre des restes de méthionine et/ou des restes d'arginine de la séquence de cardiodilatine, et/ou des fragments N-terminaux pouvant être obtenus par clivage avec une endopeptidase spécifique de l'arginine.

2. Fragment de cardiodilatine selon la revendication 1, comportant la séquence d'amino-acides :

Asp-Phe-Lys-Asn-Leu-Leu-Asp-His-Leu-Glu-Asp-Lys-Hse.

3. Fragment de cardiodilatine selon la revendication 1, comportant la séquence d'amino-acides :

Pro-Leu-Glu-Asp-Glu-Ala-Hse.

4. Fragment N-terminal de cardiodilatine

Asn-Pro-Val-Tyr-Gly-Ser-Val-Ser-Asn-Ala-Asp-Leu-Hse.

5. Procédé de préparation de l'hormone peptidique cardiodilatine, caractérisé en ce qu'on utilise un extrait d'atrium comme produit de départ et on fractionne ce produit, extractible avec des solvants aqueux, selon des procédés biochimiques usuels de purification, en utilisant un test dans lequel la fraction active est déterminée au moyen de l'effet relaxant sur la musculature lisse, ainsi que par la mesure de la conductibilité de l'eau dégagée, à travers la peau, par hydrométrie.

6. Procédé selon la revendication 5, caractérisé en ce qu'on extrait le produit d'atrium bouilli avec un acide dilué.

7. Procédé selon la revendication 5, caractérisé en ce qu'on fait adsorber l'extrait acide sur de l'acide alginique, on élue les peptides avec un acide minéral dilué, on tamponne l'éluat avec de l'acétate de sodium, on le neutralise, on le soumet à une précipitation par un sel, on dissout le précipité et on soumet le produit de dissolution à une précipitation à l'éthanol, on dissout de nouveau le précipité éthanolique et on chromatographie le produit de dissolution sur de la carboxyméthylcellulose.

8. Procédé selon la revendication 7, caractérisé en ce que le précipité obtenu par précipitation par un sel est directement débarrassé de son sel et purifié par chromatographie sur gel, de préférence sur Sephadex G 25.

9. Procédé selon la revendication 5 ou 8, caractérisé en ce qu'on prépare l'extrait d'atrium avec de l'acide acétique.

10. Procédé selon l'une des revendications 6 à 8, caractérisé en ce que, pour la purification poussée, on effectue une chromatographie en phase liquide à haute pression (HPLC) sur un gel de silice en phases inversées (élution par gradient : 0,1 % d'acide trifluoroacétique dans de l'eau contre de l'acétonitrile, 0 à 60 %), et on sépare les fractions actives.

11. Procédé selon l'une des revendications 6 à 8, caractérisé en ce que, pour la purification poussée, on effectue une chromatographie d'immunoadsorption, de préférence en utilisant des anticorps immobilisés dirigés contre des fragments de cardiodilatine selon la revendication 1.

12. Procédé selon la revendication 10, caractérisé en ce qu'on fait précéder la HPLC sur un gel de silice en phases inversées d'une HPLC sur une colonne échangeuse d'ions et/ou on recommence une ou plusieurs fois la HPLC sur gel de silice en phases inversées.

13. Médicament, notamment pour le diagnostic et le traitement de l'hypertonie et pour des opérations vasculaires et cardiaques, et le traitement de l'hypertonie, des maladies vasculaires et cardiaques après des opérations vasculaires et cardiaques, ainsi que des maladies de la peau s'accompagnant de troubles de la sécrétion sudorale, du choc cardio-vasculaire, des maladies des reins et des glandes corticosurrénales et des maladies du tube digestif, en particulier des troubles de la motilité, caractérisé en ce qu'il contient, comme principe actif, une substance selon l'une des revendications 1 à 5 conjointement avec un véhicule et/ou un diluant pharmacologiquement acceptables.

14. Médicament selon la revendication 13, caractérisé en ce qu'il contient de 10 à 1000 ng de cardiodilatine par dose unitaire.

15. Procédé pour la détermination spécifique de la cardiodilatine, caractérisé en ce qu'on travaille d'après les principes de l'essai immunologique et on utilise un anticorps qui est dirigé contre la cardiodilatine selon la revendication 1 ou contre un des fragments selon les revendications 2 à 5.
